# EUROPÄISCHE PATENTSCHRIFT

(11) **EP 2 015 675 B1**
(45) Veröffentlichungstag und Bekanntmachung des Hinweises auf die Patenterteilung: **26.11.2014**
(21) Anmeldenummer: 07722295.8
(22) Anmeldetag: 25.04.2007
(51) Int. Cl.: A61B 5/107, A61B 5/103

(54) **ANZEIGESYSTEM UND VERFAHREN ZUR DARSTELLUNG VON BODENREAKTIONSKRÄFTEN AUF EINEM MENSCHLICHEN KÖRPER**
INDICATOR SYSTEM AND METHOD FOR VISUALIZING GROUND REACTION FORCES ON A HUMAN BODY
SYSTÈME D'AFFICHAGE ET PROCÉDÉ POUR AFFICHER DES FORCES DE RÉACTION DU SOL SUR UN CORPS HUMAIN

(30) Priorität: 09.05.2006 DE 102006021788
(43) Veröffentlichungstag der Anmeldung: 21.01.2009
(73) Patentinhaber: Otto Bock HealthCare GmbH, 37115 Duderstadt (DE)
(72) Erfinder: PUSCH, Martin, 37115 Duderstadt (DE); KALTENBORN, Sven, 37115 Duderstadt (DE); ZARLING, Sven, 37115 Duderstadt (DE)
(74) Vertreter: Stornebel, Kai
(86) Internationale Anmeldenummer: PCT/DE2007/000737
(87) Internationale Veröffentlichungsnummer: WO 2007/128266

(56) Entgegenhaltungen:
- EP-A- 0 663 181
- EP-A- 1 454 584
- WO-A-02/059554
- DE-A1- 10 008 059
- DE-B3-102004 046 329
- GB-A- 2 186 375
- UA-C2- 54 139
- US-A- 4 598 717

## Beschreibung

Die Erfindung betrifft ein Anzeigesystem für Bodenreaktionskräfte eines Menschen mit einer Messplatte, die zumindest eine Sensoreinrichtung zur Erfassung von Bodenreaktionskräften aufweist, die mit einer Auswerteeinrichtung und einer Projektionseinrichtung verbunden ist, sowie ein Verfahren zur Darstellung von Bodenreaktionskräften auf einem menschlichen Körper. Das Anzeigesystem und das Verfahren sind insbesondere zur Vermessung des menschlichen Körpers und/oder prothetischer oder orthetischer Passteile in ihrer relativen Position zum menschlichen Körper und zur verbesserten Einstellung eines Prothesenaufbaues geeignet.

Der Erfolg einer prothetischen Versorgung eines Patienten hängt neben der richtigen Auswahl und Anpassung der Prothese zu einem großen Teil von der Mitwirkung des Patienten sowie dessen Akzeptanz der Prothese ab. Eine technisch ausgeklügelte Prothese erfüllt ihren Zweck nicht, wenn sie von dem Prothesenträger nicht angenommen wird oder falsch eingesetzt wird. Wichtig ist unter anderem das Verständnis für den Aufbau und die Funktionsweise der jeweiligen Prothese sowie die Kenntnis von Konsequenzen einer falschen Handhabung oder Einstellung. Darüber hinaus müssen die jeweiligen Prothesen an die individuellen Bedürfnisse und Gegebenheiten des Prothesenträgers angepasst werden. Dazu ist es wichtig, einen für den Patienten optimalen Prothesenaufbau zu ermitteln, also die Einstellung der Prothesenkomponenten zueinander sowie zu dem menschlichen Körper, um beispielsweise einen hinreichend stabilen Aufbau oder eine hohe dynamische Beweglichkeit erreichen zu können. Insbesondere bei Knieprothesen ist ein individuell angepasster Prothesenaufbau wesentlich.

Aus der EP 0 663 181 A2 ist ein Anzeigesystem bekannt, bei dem ein Prothesenträger auf einer Messplatte steht, die mit Drucksensoren bestückt ist. Über die Drucksensoren wird der Körperschwerpunkt des Prothesenträgers ermittelt. Über eine Laserprojektionseinheit, die einen linienförmigen Lichtstrahl lotrecht erzeugt, wird die Körperschwerkraftlinie auf den menschlichen Körper projiziert. Die Lage der Köperschwerkraftlinie wird durch Verfahren der Laserprojektionseinheit entlang einer Linearführung eingestellt.

Mit diesem System kann sehr gut die Schwerpunktlinie des Körpers in Bezug zu den Gelenkpositionen ermittelt werden, ebenfalls können Messdaten auf umgekehrtem Wege ermittelt werden, indem bestimmte Gelenke durch die Projektionslinie angefahren werden, so dass sich aus dem Verfahrweg der Laserprojektionseinheit ein entsprechendes Maß bestimmbar ist.

Die WO 2002/059554 A2 beschreibt ein Verfahren und eine Vorrichtung zum Überwachen von Kräften, die von einer Person ausgeübt werden, die auf einer Plattform besteht. Als Kräfte können Zugkräfte, Drehmomente oder Zugspannungen auftreten. Das Verfahren ist insbesondere dazu geeignet, um direkt diejenigen Kräfte zu ermitteln, die während der Geburt auf ein Kind ausgeübt werden. Anzeigeeinrichtungen sind vorgesehen, um die Größe der Kräfte und/oder die Richtung der auf das Kind aufgebrachten Kräfte anzuzeigen. Alarmeinrichtungen sind vorgesehen, die auf unterschiedliche Art und Weise verdeutlichen, wenn ein vorgegebener Grenzwert überschritten wird.

US 4,598,717 beschreibt eine Vorrichtung zur klinischen Dokumentation von Haltungsfehlern, insbesondere von Wirbelsäulendeformationen. Eine Plattform ist mit einer Vielzahl von Sensoren ausgestattet, die mit einem Analog-Digital-Konverter verbunden sind. Eine Kamera ist so angeordnet, dass sie ein Bild der auf der Plattform befindlichen Person aufnimmt. Ein Kraftvektor wird errechnet und dem Kamerabild überlagert.

Die DE 100 08 059 A1 beschreibt eine Anordnung und ein Verfahren zum Vermessen eines wenigstens in Teilbereichen dreidimensionalen Objektes. Ein Objektbereich wird mit einer Vermessungseinrichtung abgetastet. Ein weiterer Objektbereich wird in eine wenigstens temporär verformbare Form abgebildet und die abgebildete Form wird mit der Vermessungseinrichtung oder einer weiteren Vermessungseinrichtung abgetastet. Das Verfahren und die Anordnung sind insbesondere zum Vermessen von Körpern vorgesehen, damit Bekleidung an den jeweiligen Träger angepasst werden kann.

Die Einstellung des Prothesenaufbaus aufgrund der ermittelten Körperschwerkraftlinie wird durch einen Orthopädiemechaniker ausgeführt und bedarf einiger Erfahrung. Der Patient weiß mitunter nicht, warum welche Einstellung gewählt wird. Dies kann zu Akzeptanzproblemen führen.

Die UA 54 139 C2 betrifft ein Gerät zur Berechnung zur Standfähigkeit eines Menschen, die zur Untersuchung von Patienten mit Bewegungsapparatserkrankungen und für Berechnungen in der Prothetik verwendet werden kann. Zwei Testplattformen sind mit einem Computer, einem Steuer- und Anzeigeblock sowie einem Laserprojektor verbunden. An jeder Lagerstelle der Testplattform sind Sensoren angeordnet, die als Dehnmessstreifen ausgebildet sind. Der Patient stellt sich zur Benutzung auf die Testplattformen, über die Dehnmessstreifen werden die jeweiligen Gewichtsverteilungen erfasst und an einen Verstärker übermittelt. Aus sämtlichen Schwerkraftsdaten wird die Gesamtschwerpunktslinie ermittelt, die auf den auf beiden Testplattformen stehenden Patienten projiziert werden.

Die GB 2 186 375 A betrifft ein Biofeedback-System, das mehrere Kraftmessplatten aufweist, über die eine Gewichtsverteilung sowohl in Frontalebene als auch in Sagittalebene angezeigt werden kann. Die Anzeige erfolgt unmittelbar auf einem Display oder über ein Spiegelsystem.

Aufgabe der vorliegenden Erfindung ist es, ein verbessertes Anzeigesystem und ein Verfahren zur Darstellung von Bodenreaktionskräften bereitzustellen, mit denen eine bessere Akzeptanz und eine bessere Einstellung des Prothesenaufbaues erfolgen können.

Erfindungsgemäß wird diese Aufgabe durch ein Anzeigesystem mit den Merkmalen des Anspruchs 1 sowie ein Verfahren mit den Merkmalen des Anspruchs 6 gelöst. Vorteilhafte Ausgestaltungen und Weiterbildungen der Erfindung sind in den Unteransprüchen beschrieben.

Das erfindungsgemäße Anzeigesystem mit einer Messplatte, auf der ein Patient mit nur einem Fuß steht, die zumindest eine Sensoreinrichtung zur Aufnahme von Bodenreaktionskräften aufweist, die mit einer Auswerteeinrichtung verbunden ist, über die die von der Sensoreinrichtung ermittelten Sensordaten ausgewertet werden, wobei eine Projektionseinrichtung die ausgewerteten Daten verwendet, um einen Lichtstreifen auf einen menschlichen Körper zur projizieren, sieht vor, dass die Sensoreinrichtung als ein Mehrkomponentenkraftsensor ausgebildet sind, der die Vertikal- und Horizontalkomponenten der Bodenreaktionskraft erfasst und die Projektionseinrichtung zur Anzeige sowohl des Ortes als auch der Orientierung der erfassten Bodenreaktionskraft ausgebildet ist. Während die aus dem Stand der Technik bekannte Anzeigeeinrichtung nur den Schwerpunkt des Körpers auf der Messplatte ermittelt, ist es mit dem erfindungsgemäßen Anzeigesystem möglich, auch Horizontalkräfte zu erfassen, die durch einen Prothesenaufbau oder bei statischen Kraftmessungen oder dynamischen Kraftmessungen entstehen. Das Anzeigesystem ermöglicht die Anzeige des Kraftvektors, der tatsächlich auf die Prothese bzw. den Stumpf des Prothesenträgers wirkt, wobei der Kraftverlauf unmittelbar am Patienten dargestellt werden kann. Dadurch ist es möglich, dem Patienten unmittelbar zu verdeutlichen, welche Auswirkungen ein veränderter Prothesenaufbau auf die Kraftrichtung und dadurch auch auf die Belastung des Stumpfes hat. Eine Einstellung des Prothesenaufbaus kann somit mit verbesserter Akzeptanz durch den Prothesennutzer erfolgen. Ebenfalls ist die Einstellung des Prothesenaufbaus für den Orthopädiemechaniker einfacher als bisher, da Veränderungen sofort am Patienten sichtbar werden.

Eine Weiterbildung der Erfindung sieht vor, dass die Projektioriseinrichtung als Videoprojektor bzw. Datenprojektor oder Beamer ausgebildet ist. Über die Auswerteeinheit wird ein Lichtbalken erzeugt, der entsprechend der Orientierung des Bodenreaktionskraftvektors gedreht bzw. gekippt werden kann. Dazu muss lediglich anhand der ausgewerteten Sensordaten ein entsprechendes Videobild in Gestalt eines Lichtstreifens erzeugt werden. Dies erfordert einen geringen apparativen Aufwand. Alternativ dazu kann eine Laserprojektionseinheit, wie sie aus dem Stand der Technik bekannt ist, über einen Motor, insbesondere Schrittmotor kippbar ausgebildet sein, so dass die Orientierung des Bodenreaktionskraftvektors auf dem Patienten abgebildet werden kann. Während bei einem Datenprojektor auf einfach Art und Weise die Orientierung und die Breite des Lichtbalkens variiert werden kann, hat eine Laserprojektionseinheit ein in der Regel schärfer konturiertes Lichtbild. Die Darstellung über einen Datenprojektor oder Beamer hat den Vorteil, dass Zusatzinformationen eingeblendet werden können, beispielsweise die Größe der Kraft in Newton oder Korrekturhinweise, die aus der Auswerteeinheit mit gegebenenfalls hinterlegten Berechnungsvorschriften generiert werden.

Eine Weiterbildung der Erfindung sieht vor, dass eine Videokamera der Messplatte zugeordnet ist, so dass gleichzeitig zu der Kraftmessung über Mehrkomponentenkraftsensoren die Stellung bzw. Bewegung des Patienten aufgenommen werden kann, Durch einen kombinierten Einsatz der Projektionseinrichtung und der Videokamera ist es möglich, neben einer statischen auch eine dynamische Betrachtung des Kraftverlaufs durchzuführen. Ebenfalls ermöglicht die Videoaufzeichnung eine spätere Auswertung des Kraftverlaufes im Detail, so dass über verschiedene Bewegungsphasen oder Stellungen stets ein optimaler Prothesenaufbau eingestellt werden kann.

Zur Vermeidung von Parallaxenfehlern, die insbesondere bei kleinen Abständen zwischen der Projektionseinrichtung und dem Patienten auftreten, muss entweder ein ausreichend großer Abstand oder eine dicht nebeneinander angeordnete Kombination der Projektionseinrichtung und der Videokamera vorgesehen werden. Dabei ist es vorteilhaft, wenn die Videokamera und die Projektionseinrichtung in einer gemeinsamen optischen Ebene oder auf einer gemeinsamen optischen Achse angeordnet sind, bevorzugt mit einer im Wesentlichen gleichen Entfernung zu dem Patienten.

Das erfindungsgemäße Verfahren zur Darstellung von Bodenreaktionskräften, insbesondere zur Einstellung eines Prothesenaufbaues sieht vor, dass über die Messplatte die Bodenreaktionskräfte eines nur mit einem Fuß auf der Messplatte stehenden Patienten sowohl in horizontaler als auch in vertikaler Richtung gemessen werden. Der Angriffspunkt und die Orientierung des Kraftvektors der Bodenreaktionskraft werden auf den menschlichen Körper projiziert, so dass die Bodenreaktionskraft, der Ort und die Richtung an dem Patienten visualisiert werden kann.

Über eine Längenmodulation des Lichtbalkens, der den Bodenreaktionskraftvektor darstellt, kann der Betrag der Kraft angezeigt werden. Bei größeren Kräften entsprechend länger als bei kleineren Kräften. Für eine dynamische Betrachtung, insbesondere für eine wiederholte dynamische Betrachtung ist es vorgesehen, dass der während der Messung der Bodenreaktionskräfte auf den menschlichen Körper projizierte Bodenreaktionskraftvektor über eine Videokamera aufgenommen wird. Somit ist möglich, eine dynamische Anzeige des Bodenreaktionskraftvektorverlaufes wiederholt zu analysieren. Bei einer Verwendung eines Datenprojektors ist es möglich, sowohl die Videobilder als auch die Kraftvektorprojektion über den Datenprojektor anzuzeigen, so dass kein gesonderter Bildschirm vorhanden sein muss.

Eine Weiterbildung der Erfindung sieht vor, dass der Kraftvektor farblich dargestellt oder moduliert wird, beispielsweise wenn bestimmte Richtwerte über oder unterschritten werden. Auch kann eine geometrische Modulation vorgesehen sein. Eine belastungsabhängige Farbveränderung oder Größenveränderung kann dem Prothesennutzer oder Orthopädiemechaniker unmittelbar Hinweis über einen günstigen oder ungünstigen Prothesenaufbau geben.

Nachfolgend werden Ausführungsbeispiele der Erfindung anhand der beigefügten Figuren näher erläutert. Es zeigen:
- Figur 1 -: schematische Draufsicht eines Anzeigesystem in einer Erstausgestaltung;
- Figur 2 -: schematische Darstellung eines Anzeigesystems in einer zweiten Ausgestaltung;
- Figur 3 -: Anwendung des Systems nach Figur 2;
- Figur 4 -: alternative Darstellung der Figur 3;
- Figur 5 -: schematische Darstellung einer Projektion einer Bodenreaktionskraft;
- Figur 6 -: eine erste Ausführungsform einer Messplatte; sowie
- Figur 7 -: eine Variante der Figur 6.

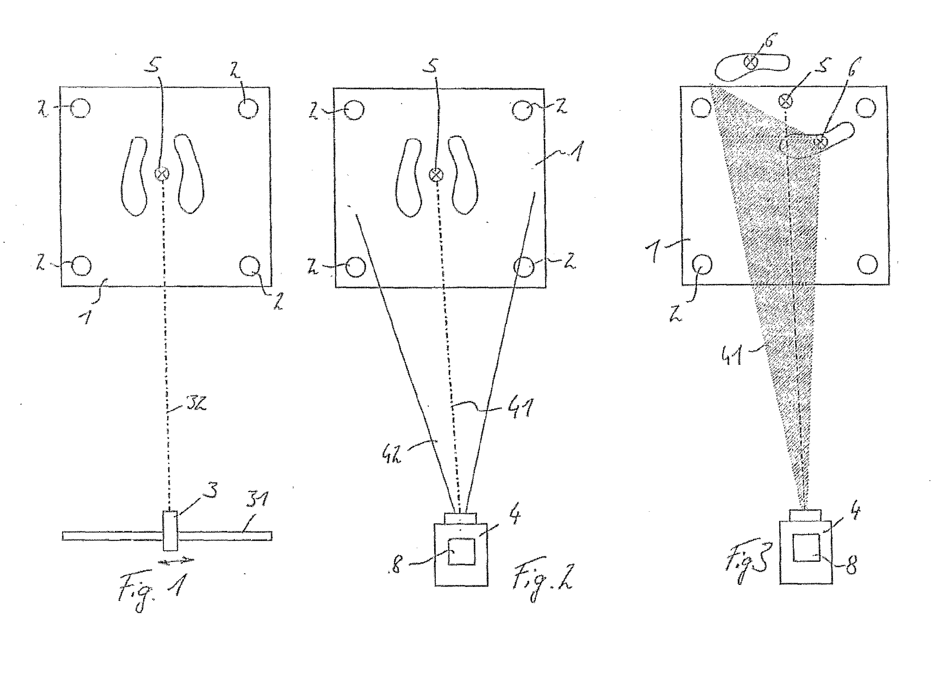

In der Figur 1 ist in Draufsicht ein Anzeigesystem zur Darstellung von Reaktionskräften mit einer Messplatte 1 gezeigt, die über Kraftsensoren, die vorliegend an den Vierecken der Messplatte 1 angeordnet sind, auf dem Boden gelagert ist. Auf der Messplatte 1 steht ein Mensch, von dem nur die Fußstellungen angezeigt sind.

Der Messplatte 1 gegenüber ist eine Projektionseinrichtung 3 angeordnet, die vorliegend als eine Laserprojektionseinrichtung ausgebildet ist. Die Laserprojektionseinrichtung 3 ist auf einer Verfahreinrichtung 31 verfahrbar angeordnet. Die Verfahrrichtungen sind durch den Doppelfeil angedeutet. Die Kraftsensoren 2 sind mit einer nicht dargestellten Auswerteeinheit gekoppelt, über die die Lage des Körperschwerpunktes 5 der auf der Messplatte 1 befindlichen Person auf der Messplatte 1 bestimmt wird. Die Auswerteeinheit errechnet den Verfahrweg der Laserprojektionseinrichtung 3 und richtet diese so aus, dass der Laserstrahl 32, der als ein Lichtbalken ausgebildet ist, auf den Körper der auf der Messplatte 1 befindlichen Person projiziert wird. Im vorliegenden Beispiel steht die Person mit beiden Füßen auf der Messplatte 1, so dass im Wesentlichen nur vertikal wirkende Bodenreaktionskräfte durch die Kraftsensoren 2 aufgenommen werden. Dementsprechend sind der Laserstrahl 32 und der projizierte Lichtbalken im Wesentlichen lotrecht ausgerichtet.

Sofern jedoch Horizontalkräfte gemessen werden, sieht das erfindungsgemäße Anzeigesystem vor, dass die Projektionseinrichtung 3 dergestalt verschwenkt wird, dass neben dem Ort des Kraftangriffspunktes der Bodenreaktionskraft auch die Orientierung angezeigt wird. Dazu muss der Laserstrahl 32 bzw. die Projektionseinrichtung 3 um eine Achse parallel zu dem dargestellten Laserstrahl 32 gedreht bzw. gekippt werden.

Eine alternative Ausgestaltung des Anzeigesystems ist in der Figur 2 dargestellt, in der statt eines verfahrbar gelagerten Laserprojektors 3 ein Datenprojektor 4 vorgesehen ist, der einen Lichtstrahl 41 innerhalb des Abstrahlbereiches 42 des Datenprojektors 4, auch Beamer genannt, auf den auf der Messplatte 1 befindlichen Menschen projiziert. Wie in der Figur 1 ist bei einer stehenden Person eine im Wesentlichen lotrechte Orientierung des Lichtbalkens 41 zu erwarten. Dieser ist im Körperschwerpunkt 5 angeordnet. Oberhalb des Datenprojektors 4, bevorzugt auf dessen optischer Achse, ist eine Videokamera 8 installiert, über die bei Messung der Bodenreaktionskraft die Projektion des Lichtbalkens 41 aufgenommen werden kann. Sofern die Videokamera 8 in einer optischen Ebene mit dem Datenprojektor 4 liegt, bevorzugt übereinander und mit dem gleichen optisch wirksamen Abstand, tritt kein Parallaxenfehler auf, so dass die Anzeige hinreichend genau ist.

In der Figur 3 ist das Anzeigesystem gemäß Figur 2 dargestellt, bei der die Person nur mit einem Fuß auf der Messplatte 1 steht. Die Kraftanleitungspunkte 6 über die Füße, gegebenenfalls Prothesenfüße, befinden sich somit einmal auf und einmal neben der Messplatte 1. Aufgrund einer gegebenenfalls wirksamen Horizontalkraft befindet sich der Lichtstrahl 41, der von dem Datenprojektor 4 auf den Körperschwerpunkt 5 projiziert wird, nicht mehr in der Lotrechten, sondern ist ausgehend von dem Krafteinleitungspunkt 6 auf der Messplatte 1 entsprechend der Orientierung des Bodenreaktionskraftvektors gekippt, so dass die Orientierung des Bodenreaktionskraftvektors angezeigt und auf den Körper projiziert wird. Diese Projektion kann über die Videokamera 8 aufgenommen werden. So ist es möglich, einen gehenden Menschen aufzunehmen, wobei ein Fuß auf der Messplatte 1 und ein anderer daneben aufgesetzt ist. Im Verlauf des Schrittes bzw. der Schritte können dann die Bodenreaktionskräfte auf den menschlichen Körper projiziert und eine dynamische Betrachtung durch das Videobild wiederholt und im Detail erfolgen. Die Wiedergabe des Videobildes, das durch die Videokamera 8 aufgenommen wurde, kann durch den Beamer 4 erfolgen, gegebenenfalls auf einer Leinwand oder auf ein hinter der Messplatte 1 befindlichen Wand.

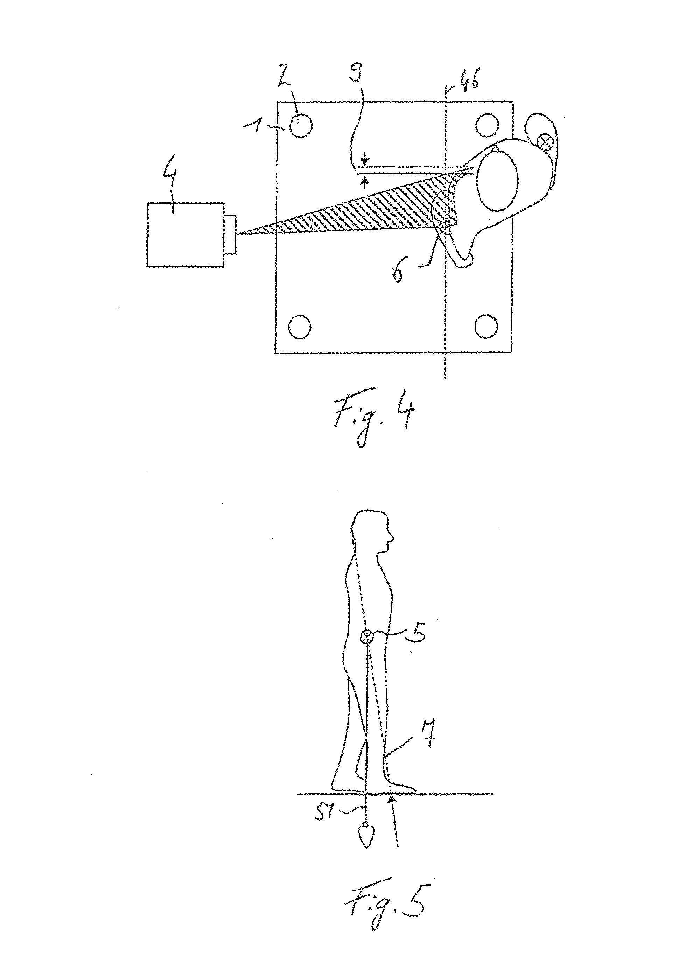

In der Figur 4 ist das Anzeigesystem gemäß Figur 3 dargestellt, bei dem der Lichtstrahl 41, der den Bodenreaktionskraftvektor darstellt, auf eine Person projiziert wird. Die Projektion kann dabei so erfolgen, dass der Lichtstrahl geometrisch oder farblich moduliert wird, um anzuzeigen, wie groß der Betrag des Bodenreaktionskraftvektors ist oder ob sich die Bodenreaktionskräfte innerhalb eines akzeptablen bzw. vorgegebenen Rahmen befinden. Dadurch kann ermittelt werden, ob eine Prothese korrekt eingestellt ist, also ob ein günstiger Prothesenaufbau vorliegt. Darüber hinaus ist es möglich, an der Prothese Veränderungen vorzunehmen, während der Bodenreaktionskraftvektor auf die Person projiziert wird, die auf der Messplatte 1 steht. Der Lichtbalken 41 oder der Laserstrahl wird dann während der Veränderung des Prothesenaufbaues in dem Maße verkippt, wie sich die Bodenreaktionskraft verändert. Vorteilhafterweise schaut der Orthopädiemechaniker dabei aus der Richtung des Projektors 4, um Parallaxenfehler möglichst zu vermeiden.

In der Figur 4 ist ein Projektionsfehler 9 dargestellt, der aufgrund der nicht ebenen Projektionsfläche auf den menschlichen Köper entsteht; die rechnerisch ermittelte und optisch korrekte Projektionsfläche 46 liegt in der Ebene senkrecht zu der Messplatte 1 und geht durch den Krafteinleitungspunkt 6 hindurch. Dieser Projektionsfehler 9 ist in der Regel gering und kann vernachlässigt werden. Die Längenmodulation kann über den Strahlensatz und die Kenntnis des Krafteinleitungspunktes 6 aufgrund der verschiedenen Kraftsensoren 2 und deren Auswertung errechnet werden.

Die Figur 5 zeigt eine Seitenansicht eines Patienten mit dem Körperschwerpunkt 5, der Körperschwerpunktlinie 51 als Lotrechte sowie der Bodenreaktionskraft 7, die in einem Winkel zu der Horizontalen ausgerichtet ist. Entlang der Orientierung der Bodeneaktionskraft 7 wird der Lichtbalken projiziert.

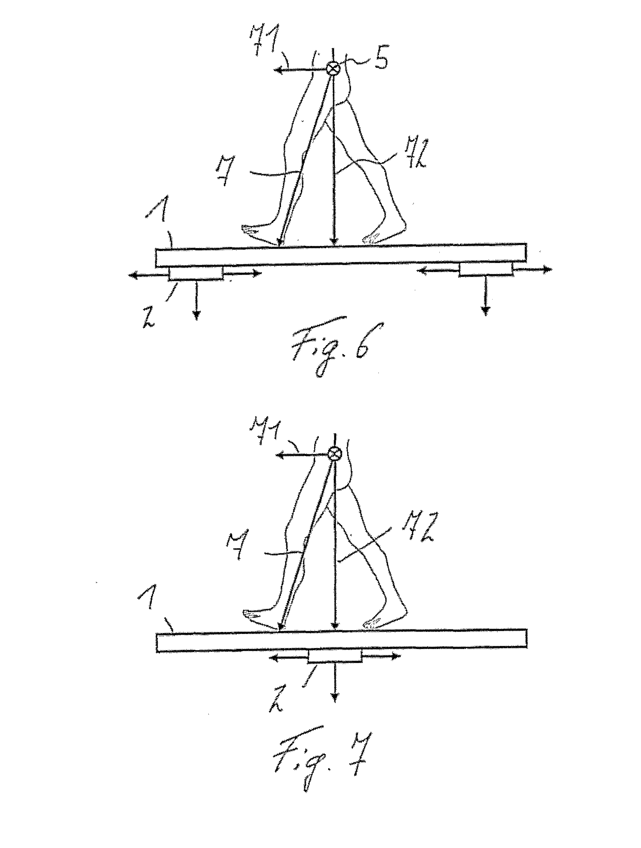

Die Figuren 6 und 7 zeigen verschiedene Konstruktionen der Messplatte 1 und die Zusammensetzung der Bodenreaktionskraft 7 aus einer Vertikalkomponenten 72 und einer Horizontalkomponente 71, die im Körperschwerpunkt 5 angreifen. Die Errechnung der Bodenreaktionskraft 7 und die Bestimmung der Orientierung erfolgt über die Messung der Vertikalkomponente 72 und Horizontalkomponente 71 über die Kraftsensoren 2, die als Mehrkomponentenkraftsensoren ausgebildet sind. Gemäß Figur 6 können mehrere Kraftsensoren 2 vorzugsweise an den Ecken der Messplatte 1 angeordnet sein, gemäß Figur 7 ist ein Mehrkomponentenkraftsensor 2 zentral an der Messplatte 1 angeordnet; alle Kraftsensoren 2 ermitteln die vertikalen und horizontalen Kräfte während der statischen oder dynamischen Belastung. Dadurch ist eine statische und dynamische Betrachtung des Kraftverlaufes möglich, der während des Stehens oder Gehens auf den Patienten und die Prothese einwirkt. Zusätzlich zu dem Lichtbalken bzw. dem Laserstrahl ist es bei der Verwendung eines Datenprojektors 4 möglich, Zusatzinformationen wie den Betrag der jeweiligen Komponenten oder Verstellvorschläge einzublenden.

## Patentansprüche

1. Anzeigesystem zur Darstellung von Bodenreaktionskräften eines nur mit einem Fuß auf einer Messplatte (1) stehenden Menschen bei der Einstellung eines Prothesen- oder Orthesenaufbaues, wobei die Messplatte (1) zumindest eine Sensoreinrichtung (2) aufweist, mit einer Auswerteeinrichtung zur Auswertung der Sensorsignale sowie eine Projektionseinrichtung (3, 4) zur Visualisierung erfasster Sensordaten, **dadurch gekennzeichnet, dass** die Sensoreinrichtung (2) als Mehrkomponentenkraftsensor ausgebildet ist, der Vertikal- und Horizontalkomponenten (71, 72) einer Bodenreaktionskraft (7) erfasst und die Projektionseinrichtung (3, 4) zur Anzeige des Ortes und der Orientierung der Bodenreaktionskraft (7) auf dem Menschen ausgebildet ist.

2. Anzeigesystem nach Anspruch 1, **dadurch gekennzeichnet, dass** die Projektionseinrichtung als Datenprojektor (4), insbesondere als Videoprojektor oder als verfahrbare und kippbare Laserprojektionseinheit (3) ausgebildet ist.

3. Anzeigesystem nach Anspruch 1 oder 2, **dadurch gekennzeichnet, dass** eine Videokamera (8) der Messplatte (1) zugeordnet ist.

4. Anzeigesystem nach Anspruch 3, **dadurch gekennzeichnet, dass** die Projektionseinrichtung (3, 4) und die Videokamera (8) unmittelbar nebeneinander angeordnet sind.

5. Anzeigesystem nach Anspruch 3 oder 4, **dadurch gekennzeichnet, dass** die Videokamera (8) und die Projektionseinrichtung (3, 4) in einer gemeinsamen optischen Ebene angeordnet sind.

6. Verfahren zur Darstellung von Bodenreaktionskräften bei der Einstellung eines Prothesen- oder Orthesenaufbaues, bei dem über eine Messplatte (1) die vertikalen und horizontalen Bodenreaktionskräfte (71, 72) einer nur mit einem Fuß auf der Messplatte (1) stehenden Person gemessen und der Angriffspunkt (6) und die Orientierung des Kraftvektors der Bodenreaktionskraft (7) auf den menschlichen Körper projiziert werden.

7. Verfahren nach Anspruch 6, **dadurch gekennzeichnet, dass** der Kraftvektor Über einen Datenprojektor (4) oder über eine verfahr- und kippbare Laserprojektionseinheit (3) auf den menschlichen Körper projiziert wird.

8. Verfahren nach Anspruch 6 oder 7, **dadurch gekennzeichnet, dass** über eine Längenmodulation der Projektion der Betrag der Bodenreaktionskraft angezeigt wird.

9. Verfahren nach einem der Ansprüche 6 bis 8, **dadurch gekennzeichnet, dass** der Bodenreaktionskraftvektor auf einen Menschen projiziert wird und diese Projektion während der Messung der Bodenreaktionskraft über eine Videokamera (8) aufgenommen und in einer Videobilddarstellung angezeigt wird.

10. Verfahren nach einem der Ansprüche 6 bis 9, **dadurch gekennzeichnet, dass** eine dynamische Anzeige des Bodenreaktionskraftverlaufs erfolgt.

11. Verfahren nach einem der Ansprüche 6 bis 10, **dadurch gekennzeichnet, dass** die Projektion des Bodenreaktionskraftvektors in Abhängigkeit der Orientierung und/oder dem Betrag des Bodenreaktionskraftvektors farblich oder geometrisch moduliert wird.

## Claims

1. A display system for displaying ground reaction forces of a human being standing with only one foot on a measurement plate (1) during adjustment of a set-up of a prosthesis or orthosis, whereas the measurement plate (1) comprises at least one sensor device (2), with an evaluation device for evaluating the sensor signals and a projection device (3, 4) for visualizing detected sensor data, **characterized in that** the sensor device (2) is in the form of a multi-component force sensor which detects vertical and horizontal components (71, 72) of a ground reaction force and the projection device (3, 4) is designed to display the location and orientation of the ground reaction force (7) on the human being.

2. The display system as claimed in claim 1, **characterized in that** the projection device is in the form of a data projector (4), in particular in the form of a video projector or a moveable laser projection unit (3) which can be tilted.

3. The display system as claimed in claim 1 or 2, **characterized in that** a video camera (8) is associated with the measurement plate (1).

4. The display system as claimed in claim 3, **characterized in that** the projection device (3, 4) and the video camera (8) are arranged directly adjacent to one another.

5. The display system as claimed in claim 3 or 4, **characterized in that** the video camera (8) and the projection device (3, 4) are arranged on a common optical plane.

6. A method for displaying ground reaction forces during adjusting a prosthesis set-up or orthosis set-up, in which the vertical and horizontal ground reaction forces (71, 72) of a person standing with one foot only on a measurement plate (1) are measured by the measurement plate (1) and the point of contact (6) and the orientation of the force vector of the ground reaction force (7) is projected onto the human body.

7. The method as claimed in claim 6, **characterized in that** the force vector is projected onto the human body by means of a data projector (4) or a moveable laser projection unit (3) which can be tilted.

8. The method as claimed in claim 6 or 7, **characterized in that** the magnitude of the ground reaction force is displayed by a length modulation of the projection.

9. The method as claimed in one of claims 6 to 8, **characterized in that** the vector of the ground reaction force is projected onto a human being and this projection is recorded by a video camera (8) during the measurement of the ground reaction force and is displayed in a video image display.

10. The method as claimed in one of claims 6 to 9, **characterized in that** the profile of the ground reaction force vector is displayed dynamically.

11. The method as claimed in one of claims 6 to 10, **characterized in that** the projection of the ground reaction force vector is modulated in color or geometrically depending on the orientation and/or magnitude of the ground reaction force vector.

## Revendications

1. Système d'affichage pour la représentation de forces de réaction au sol d'un être humain debout sur un seul pied sur une plaque de mesure (1) lors du réglage d'une structure de prothèse ou d'orthèse, dans lequel la plaque de mesure (1) comprend au moins un système à capteur (2), et comprenant un système d'évaluation pour l'évaluation des signaux de capteur, ainsi qu'un système de projection (3, 4) pour la visualisation des données de capteur saisies, **caractérisé en ce que** le système à capteur (2) est réalisé comme un capteur de force à multiples composantes, qui saisit des composantes verticale et horizontale (71, 72) d'une force de réaction au sol (7), et le système de projection (3, 4) est réalisé pour afficher le lieu et l'orientation de la force de réaction au sol (7) sur l'être humain.

2. Système d'affichage selon la revendication 1, **caractérisé en ce que** le système de projection est réalisé sous forme de projecteur de données (4), en particulier de projecteur vidéo ou sous forme d'unité de projection à laser (3) déplaçable en translation et en basculement.

3. Système d'affichage selon la revendication 1 ou 2, **caractérisé en ce qu'**une caméra vidéo (8) est associée à la plaque de mesure (1).

4. Système d'affichage selon la revendication 3, **caractérisé en ce que** le système de projection (3, 4) et la caméra vidéo (8) sont agencés directement l'un à côté de l'autre.

5. Système d'affichage selon la revendication 3 ou 4, **caractérisé en ce que** la caméra vidéo (8) et le système de projection (3, 4) sont agencés dans un plan optique commun.

6. Procédé pour représenter des forces de réaction au sol lors du réglage d'une structure de prothèse ou d'orthèse, dans lequel on mesure au moyen d'une plaque de mesure (1) les forces de réaction au sol verticale et horizontale (71, 72) d'une personne debout sur un seul pied sur la plaque de mesure (1), et l'on projette le point d'attaque (6) et l'orientation du vecteur de force de la force de réaction au sol (7) sur le corps humain.

7. Procédé selon la revendication 6, **caractérisé en ce que** le vecteur de force est projeté sur le corps humain au moyen d'un projecteur de données (4) ou d'une unité de projection à laser (3) déplaçable en translation et en basculement.

8. Procédé selon la revendication 6 ou 7, **caractérisé en ce que** l'on indique l'intensité de la force de réaction au sol au moyen d'une modulation en longueur de la projection.

9. Procédé selon l'une des revendications 6 à 8, **caractérisé en ce que** le vecteur de la force de réaction au sol est projeté sur un être humain et cette projection est enregistrée au moyen d'une caméra vidéo (8) pendant la mesure de la force de réaction au sol et est affichée dans une représentation d'imagerie vidéo.

10. Procédé selon l'une des revendications 6 à 9, **caractérisé en ce que** l'on procède à un affichage dynamique de l'évolution de la force de réaction au sol.

11. Procédé selon l'une des revendications 6 à 10, **caractérisé en ce que** la projection du vecteur de la force de réaction au sol est modulée soit en couleurs soit de manière géométrique en fonction de l'orientation et/ou de l'intensité du vecteur de la force de réaction au sol.
